# EUROPEAN PATENT SPECIFICATION

(11) **EP 2 049 901 B1**
(45) Date of publication and mention of the grant of the patent: **20.07.2011**
(21) Application number: 07713236.3
(22) Date of filing: 12.03.2007
(51) Int. Cl.: G01N 33/543, C12Q 1/68

(54) **METHOD FOR ANALYZING SAMPLES INCLUDING A GAS EVOLVING MEANS**
VERFAHREN ZUR ANALYSE VON PROBEN MIT EINEM MITTEL ZUR GASENTWICKLUNG
PROCÉDÉ POUR ANALYSER DES ECHANTILLONS COMPRENANT UN MOYEN DE DEGAGEMENT DE GAZ

(30) Priority: 19.07.2006 EP 06117464
(43) Date of publication of application: 22.04.2009
(73) Proprietor: Koninklijke Philips Electronics N.V., 5621 BA Eindhoven (NL)
(72) Inventor: GILLIES, Murray, Fulton, 5656 AA Eindhoven (NL); VOSSENAAR, Erik Robbert, 5656 AA Eindhoven (NL); JOHNSON, Mark Thomas, 5656 AA Eindhoven (NL); STAPERT, Hendrik Roelof, 5656 AA Eindhoven (NL); FISH, David Andrew, NL-5656 AA Eindhoven (NL); KURT, Ralph, 5656 AA Eindhoven (NL); PENTERMAN, Roel, 5656 AA Eindhoven (NL); CATTANEO, Stefano, 5656 AA Eindhoven (NL)
(74) Representative: Van Velzen, Maaike Mathilde
(86) International application number: PCT/IB2007/050808
(87) International publication number: WO 2008/010105

(56) References cited:
- WO-A-03/049677
- WO-A2-03/076937

## Description

### FIELD OF THE INVENTION

The present invention is directed to the field of devices for the detection of one or more target molecules in a fluid sample, especially to the field of devices for the detection of biomolecules in aqueous solution. Furthermore, the present invention is directed to a control circuit to simplify the analysis of the sample.

### BACKROUND OF THE INVENTION

The present invention is directed to the detection of target molecules in fluids, especially to the detection of biomolecules in aqueous solution. The detection usually occurs in such a way that the fluid to be analyzed is provided on a substrate material, which contains capture sites for the target molecules which are subject of the detection. Such a capture site may be a corresponding DNA-strand in case the target molecule is also a DNA-strand or an antibody in the case of a protein assay. The target mo lecules in the fluid will then bind to the capture site and remain there even after the fluid is removed. The target molecule contains a label and in this way may be detected.

WO03/076937A2 describes an apparatus for conducting a variety of assays for the determination of analytes in liquid samples, and relates to methods for such assays. The document relates to a single-use cartridge designed to be adaptable to a variety of real-time assay protocols, preferably assays for the determination of analytes in biological samples using immunosensors or other ligand/ ligand receptor-based biosensor embodiments. Described is a known washing step for removing unbound material by means of fluid or gases evolved by electrolyses.

However, especially if small volumes of samples are to be detected, the removal of non-bound but marked species in the sample is a problem, since these compounds must be removed in order not to cause a later misreading. In prior art, therefore excessive washing steps are common which on the one hand lengthen the analysis procedure and on the other hand add complexity and cost to the analytical device.

### SUMMARY OF THE INVENTION

It is therefore an object of the present invention to provide a method, which allows a quicker detection with a minute amount of target molecule that needs to be present in the fluid.

This object is solved by a method for analyzing one or more samples, especially fluid samples for the presence, amount or identity of one or more target molecules in the samples, comprising the steps of

Evolving gas by a gas evolving means by electrolysis of the sample solution (30) with a voltage of ≥1,2V and ≤ 10V, thereby

Washing away and/ or removing unbound target molecules and/ or redundant sample fluid from a capture site (10), wherein only the hydrogen is used within the gas evolving means.

Surprisingly it has been shown that it is for most applications within the present invention possible to remove non-bound species from the capture sites by guiding gas over them whilst bound target molecules will not be removed.

A substrate material as disclosed by the present invention has the following advantages over the prior art:
- The removal of non-bound species is efficient and time-effective, thus reducing the amount of time needed for the analysis
- No washing agent or associated microfluidics / reservoir are required
- The outcoupling of fluorescent light is improved as no light is guided away from the detector through the sample liquid

As will be appreciated by those in the art, the sample may comprise any number of things, including, but not limited to, bodily fluids (e.g. blood, urine, serum, lymph, saliva, anal and vaginal secretions, perspiration and semen) of virtually any organism, with mammalian samples being preferred and human samples particularly preferred; environmental samples (e.g. air, agricultural, water and soil samples); biological warfare agent samples; research samples (i.e. in the case of nucleic acids).

As will be appreciated by those in the art, the target molecule(s) may be, but not limited to, the product(s) of an amplification reaction, including both target and signal amplification); purified samples, such as purified genomic DNA, RNA, proteins, etc.; raw samples (bacteria, virus, genomic DNA, etc.); biological molecular compounds such as, but not limited to, nucleic acids and related compounds (e.g. DNAs, RNAs, oligonucleotides or analogs thereof, PCR products, genomic DNA, bacterial artificial chromosomes, plasmids and the like), proteins and related compounds (e.g. polypeptides, peptides, monoclonal or polyclonal antibodies, soluble or bound receptors, transcription factors, and the like), antigens, ligands, haptens, carbohydrates and related compounds (e.g. polysaccharides, oligosaccharides and the like), cellular fragments such as membrane fragments, cellular organelles, intact cells, bacteria, viruses, protozoa, and the like.

As will be appreciated by those in the art, virtually any experimental manipulation may have been done on the sample and /or the target molecules.

The gas evolving means evolves gas by applying a voltage of ≥1,2 V and ≤10V. This has been shown to be a suitable voltage range in order to obtain suitable gas bubbles in a reproducible manner. According to a preferred embodiment of the present invention, the gas evolving means evolves gas by applying a voltage of ≥2 V and ≤4,5V, according to a preferred embodiment of the present invention, the gas evolving means evolves gas by applying a voltage of ≥2,5 V and ≤3,5V.

According to a preferred embodiment of the present invention, the gas evolving means evolves gas by using a current of ≥10⁻⁸ A and ≤ 10⁻⁶ A.

This has been shown within a wide range of applications to be a suitable voltage range in order to obtain suitable gas bubbles in a reproducible manner.

According to a preferred embodiment of the present invention, the gas evolving means evolves gas by using a current of ≥ 2,5* 10⁻⁸ A and ≤ 7,5* 10⁻⁷ A. According to a preferred embodiment of the present invention, the gas evolving means evolves gas by using a current of ≥5*10⁻⁸ A and ≤ 5*10⁻⁷ A.

According to a preferred embodiment of the present invention, the gas evolving part of the gas evolving means is located in a distance of ≥0 mm and ≤5 mm from the capture sites. This distance has been shown to be advantageous in practice for a wide range of applications within the present invention. According to a preferred embodiment of the present invention, the gas evolving part of the gas evolving means is located in a distance of ≥ 10 µm and ≤1 mm, preferably ≥ 100µm and ≤500µm from the capture sites.

It should be noted that only the hydrogen evolving part of the gas evolving means will be used whereas the oxygen evolving part is preferably located more remote from the capture sites or according to another preferred embodiment located in a separate component of the device.

In embodiments within the present invention which use a gas evolving means of this composition, it is preferred that the hydrogen evolving part of the gas evolving means is located in a distance of ≥ 0 mm and ≤5 mm from the capture sites. This distance has been shown to be advantageous in practice for a wide range of applications within the present invention. According to a preferred embodiment of the present invention, the gas evolving part of the gas evolving means is located in a distance of ≥ 10µm and ≤1 mm, preferably ≥ 100µm and ≤500µm from the capture sites.

In some applications within the present invention, oxygen may be harmful either to the capture sites, the target molecules or the labels, which are in most application fluorescent dyes some of which are known to be quite sensitive to oxygen. Therefore only the hydrogen is used within the gas evolving means whereas the oxygen is held in solution and/or evolved at a remote part of the device.

According to a preferred embodiment of the present invention, the gas evolving means comprises at least one pair of electrodes.

According to a preferred embodiment of the present invention, the gas evolving means is also adapted to serve as a means to transport molecules, especially the target molecules to the capture site. The method for transportation may according to one embodiment of the present invention be provided in the form of electrophoretic transport (if the particles are charged as is the case for DNA and proteins) or via di-electrophoresis for uncharged molecules. Such uncharged molecules can for example be beads coated with antibodies. In the latter case it is preferred that a traveling wave with four phases is used. Alternatively, pressure differences created by the local generation of gas can be used according to one embodiment of the present invention to move the solution containing the target molecules and by this create mixing and improve the chance of capturing.

Alternatively or additionally and insofar another embodiment of the present invention electro-osmotic movement may be generated to create mixing and improve the chance of capturing.

The present invention also dicloses a gas evolution control means in particular for removing unbound target molecules and/or redundant sample fluid from at least one capture site of a device according to the present invention including a feedback mechanism controlling the formation and/or the movement of evolved gas bubbles.

However, it should be noted that such a gas evolution control means is also of inventive use without such a device and may be employed in a wide range of other applications.

It is well known that the nucleation of gas bubbles from a liquid is within a wide range of applications not a well defined process and can be rather unpredictable as it depends on many device parameters such as the surface of the gas evolving means and/or whether there is significant local roughness or conductivity of the sample liquid. Furthermore, it is for a wide range of applications not possible or very difficult to allow one capture site to further hybridize, since the signal is weak, while stopping another side where the signal has reached its maximum.

Therefore it is advantageous to provide such a gas evolution control means to allow a controlled development of the gas bubbles.

The control means may include feedback mechanism to accurately control the amount and/or the formation and/or the movement of the gas bubbles. The feedback mechanism may be for example at least one active control circuit and/or may comprise at least one activating means.

The gas evolution control means may comprise at least two electrodes for said gas evolution and the feedback mechanism comprises an impedance network of at least one capacitor and /or resistor which is/are located between said electrodes to measure a capacity and/ or a resistance between said electrodes to control the formation and/or the movement of the gas bubble.

This feedback mechanism may control the amount and/or the movement of gas evolved by the gas evolving means by measuring the change for example in capacitance and/or resistance between two neighboring electrodes and comparing the value with a predetermined value.

The gas evolution control means may be adapted in such a way that upon a change of the impedance, resistance and/or the capacity a signal is initiable by said activating means to activate a third electrode.

Since the sample solvent (which in most applications will be water based) has a greatly different resistance than the gas evolved by the gas evolving means, the difference in capacity and/or resistance can be measured easily and therefore it is possible to determine very accurately how far the "gas front" has progressed along the electrodes.

In a further embodiment of the present invention the electrodes are arranged as an active matrix such as a Large Area Electronic (LAE) technologies for example LTPS so that it is possible to individually address several electrodes at each capture side.

Advantageously in such a matrix the sample fluid at each side can be evacuated at any point in time. This allows either stopping of the hybridization at the maximum signal level or alternatively creating a sequence of otherwise identical sides but where the hybridization has been stopped at different points in time and so allowing the kinetics of the hybridization to be characterized.

The present invention furthermore discloses a method for analyzing one or more samples especially fluid samples for the presence, amount or identity of one or more target molecules in the samples, comprising the steps of
(a) providing a device as described above
(b) adding an amount of sample to the substrate material, preferably to one or more defined areas on the substrate material
(c) allowing the target molecule in the samples to possibly combine with one or more of the capture sites
(d) evolving gas from the gas evolving means to remove non-bound target molecules
(e) optionally controlling and finally stopping the gas evolution via the gas evolution control means.

By doing so a quick and reliable detection of target molecules in the sample, even in minute amounts, is feasible for most applications within the present invention.

A method according to the present invention and a substrate material, a gas evolutions means end/or a device disclosed by the present invention may be of use in a broad variety of systems and/or applications, amongst them one or more of the following:
- biosensors used for molecular diagnostics
- rapid and sensitive detection of proteins and nucleic acids in complex biological mixtures such as e.g. blood or saliva
- electrolysis to create a local pH variation for cell lysing or protein manipulation
- high throughput screening devices for chemistry, pharmaceuticals or molecular biology
- testing devices e.g. for DNA or proteins e.g. in criminology, for on-site testing (in a hospital), for diagnostics in centralized laboratories or in scientific research
- tools for DNA or protein diagnostics for cardiology, infectious disease and oncology, food, and environmental diagnostics
- tools for combinatorial chemistry
- analysis devices

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

### BRIEF DESCRIPTION OF THE DRAWINGS

Additional details, characteristics and advantages of the object of the invention are disclosed in the subclaims, the figures and the following description of the respective figures and examples, which --in an exemplary fashion-- show several aspects of a device disclosed by the invention.
Fig. 1 shows a very schematic partial top view of a first device as disclosed by the present invention;
Fig. 2 shows a very schematic partial top view of a second device as disclosed by the present invention;
Fig. 3 shows a very schematic cross sectional partial view of the device of Fig. 2 somewhat along line II-II in Fig.2 prior to the evolution of gas;
Fig. 4 shows a very schematic cross sectional partial view of the device of Fig. 3 after evolution of a certain amount of gas; and
Fig.5 shows a very schematic cross sectional partial view of the device of Fig. 3 after evolution of an amount of gas high enough to remove unbound target molecules from the capture site;
Fig.6 shows schematically an active control means including a feedback mechanism as disclosed by the present invention; and
Fig. 7 shows schematically an activating means as disclosed by the present invention.

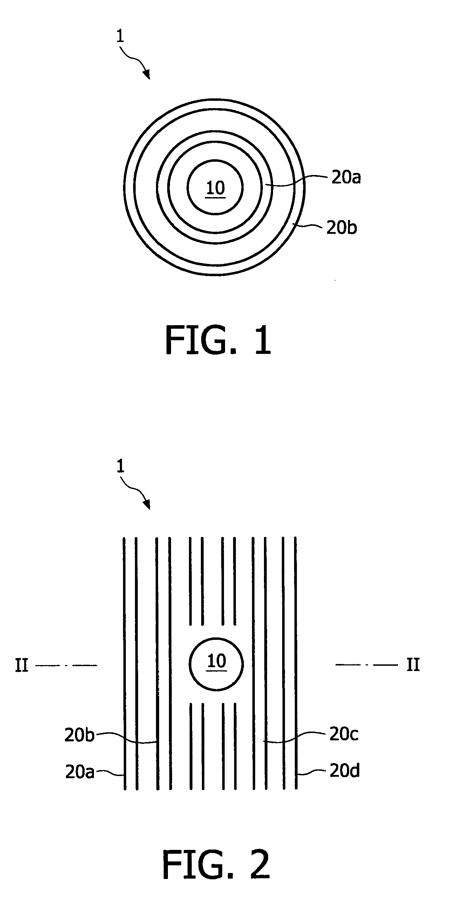

Fig. 1 shows a very schematic partial top view of a device 1 as first device . The device comprises a capture site 10 which is circumferentially surrounded by two electrodes 20a and 20b. It should be noted that the capture site 10 may indeed comprise a plurality of capture sites for several target molecules to be analyzed in the sample; however, the device may as well comprise several sets of capture sites which are all surrounded by electrodes. The number of electrodes per capture site is not restricted to two.

Fig. 2 shows a very schematic partial top view of a device 1 as selond devices. In this device, several elongated electrodes 20a, 20b, 20c and 20d are depicted. Only a few electrodes and only one capture site is shown; however, it is clear to any skilled person in the art that most applications within the present invention will use a plurality of electrodes as well as capture sites.

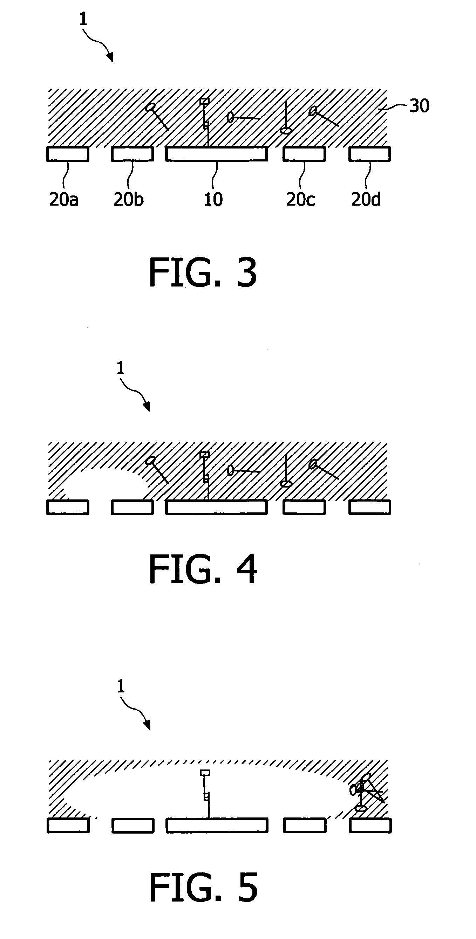

Fig. 3 shows a very schematic cross sectional partial view of the device of Fig. 2 somewhat along line II-II in Fig.2 prior to the evolution of gas. In Fig. 3 is (very schematically) shown an target molecule which is bound to the capture site 10 (e.g. via hydrogen bonds such as in nucleic acids). The target molecule is marked with a fluorescent dye (which is "indicated" by the square). However, several other nonbound target molecules are present in the sample solution 30 which might lead to misreading of the analysis when not washed away.

Fig. 4 shows a very schematic cross sectional partial view of the device of Fig. 3 after evolution of a certain amount of gas. Fig. 5 shows a very schematic cross sectional partial view of the device of Fig. 3 after evolution of an amount of gas high enough to remove unbound target molecules from the capture site.

From Fig. 4 and 5 it can be clearly seen that due to the evolution of gas via the electrodes, a gas bubble is created which removes the unbound target molecules whereas the bound target molecule will stay on the capture site 10 due to its binding.

The electrodes 20a-d can also be used to monitor the evolution of the gas bubble, since the gas (which is in this embodiment mainly hydrogen) has a greatly different resistance than the sample solution (which is in this embodiment essentially water). Therefore, by monitoring the capacity and/or the resistance between the electrodes 20a to 20d, it can be seen how the gas bubble expands and therefore the gas "front" progresses towards the electrode 20d. When this electrode 20d is reached, the gas evolution can be stopped since the unbound target molecules are far enough from the capture site 10.

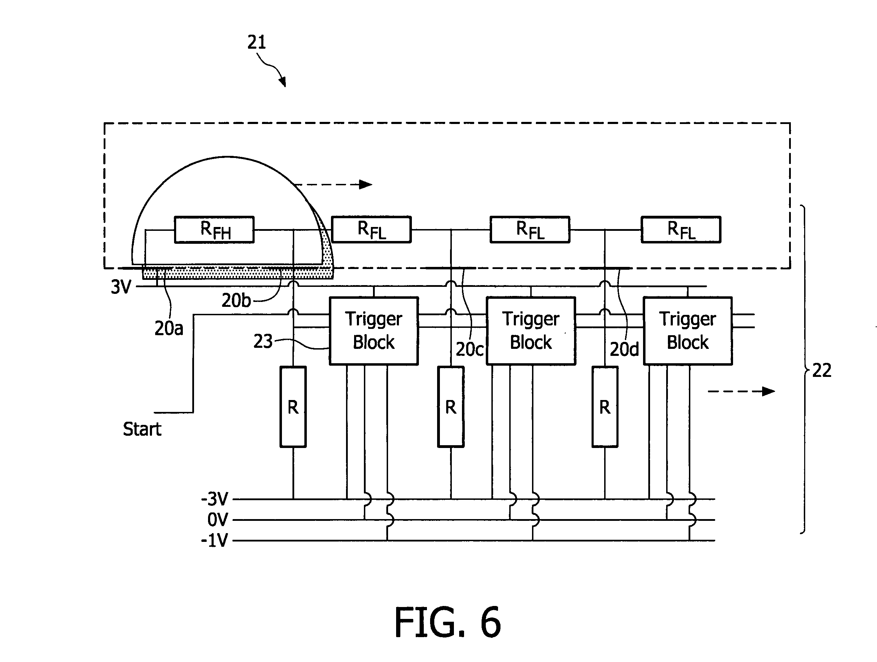

Figure 6 shows schematically an active control circuit 21 including a feedback mechanism 22 as disclosed by the present invention.

Four electrodes 20a -20d are arranged symmetrically along the capture side 10 of the device 1. Each electrode 20a-20d is connected to a resistor which serves as a reference value. An activating means 23a is assigned to compare the value of this resistor R with a value measured at the two neighboring electrodes 20a, 20b one of which is connected to another resistor R.

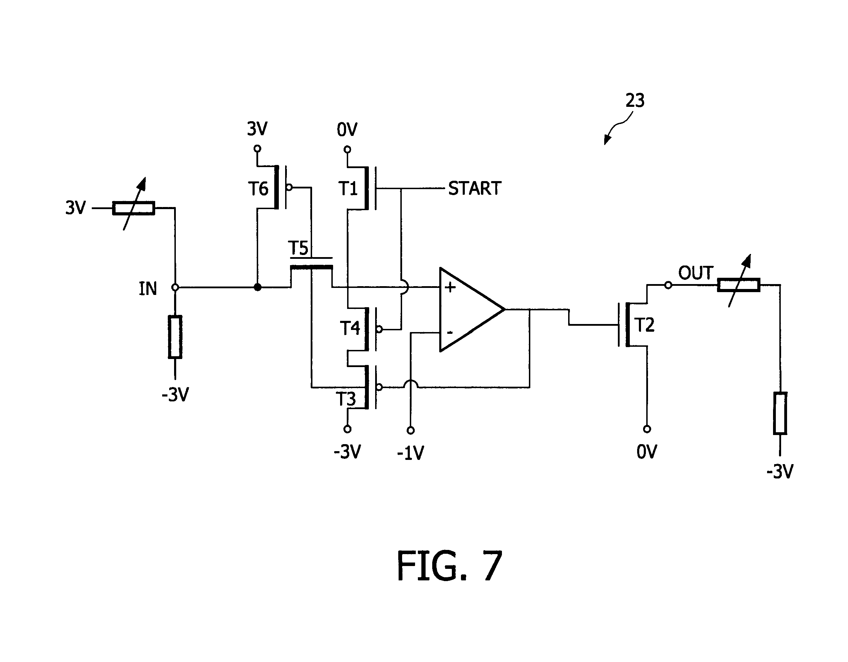

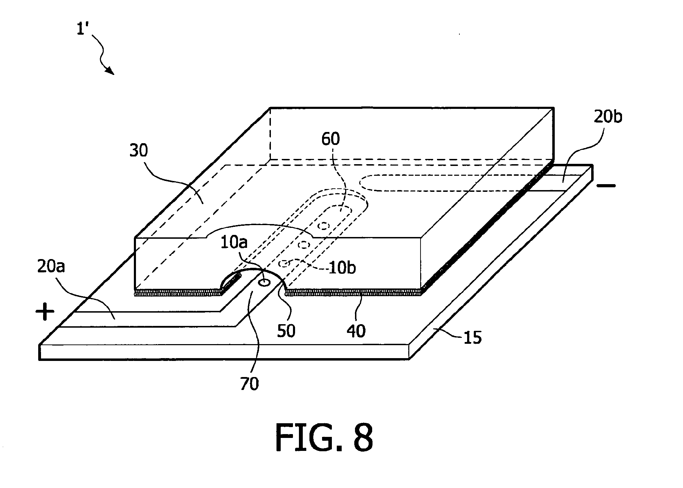

Initially there is no power supply to the circuit 21 so that no electrolysis occurs in the vicinity of the electrodes 20a - 20d. As the power supply is exemplarily moved to 3V the start line is pulsed. In this state the voltage between electrodes 20a and 20b is 3V and the potential between the other following electrodes 20c to 20n is 0V. As the start pulse goes low the voltage of the fluid resistance R_{FL} and the resistor R determines the voltage at electrode 20b. These two values are compared in the activating means 23, in this case a trigger block (e.g. Fig. 7). As long as the electrode is surrounded by the fluid of the sample there will be no great differences between these two values. As soon as the bubble front of the gas bubble evolved at the electrode 20a reaches and surrounds electrode 20b the resistance between these two electrodes increases, i.e. it changes from R_{FL} to R_{FH} the trigger block 23 measures a drop of the voltage at electrode 20b. This difference causes the trigger block 23 to fire. It then forces the electrode 20b to 3V and enables the potential divider action between the fluid resistance R_{FL} of the electrode 20b and 20c and the resistor R attached to electrode 20c, These steps repeat until the end of the electrode structure. If desired the process can be stopped anytime by initiating a high voltage at a following electrode (correct if it is not possible or if there are more possibilities to stop).

Once the resistance R_{FL} goes high the electrode20a can be automatically brought to a high voltage to stop electrolysis between two electrodes 20a and 20b and to cause electrolysis between the next two electrodes 20b and 20c and thereby enlarge the gas bubble in the desired direction. After a short period of time after the electrode 20a was shut down the gas will be absorbed by the fluid.

In some cases it may be preferred not to stop the gas evolution by shutting down the electrodes after the gas "front" has reached the next electrode, for example if the gas should be sustained to avoid a reduction of the volume occupied by the gas. This can for example be advantageous in a chamber in which the gas is utilized to move a piston forward and to hold it in this position or move it further. If on the other hand the gas is not sustained in the chamber a negative pressure can emerge and possibly move the piston oppositely.

The starting point of electrolysis may be different from time to time. Therefore in an alternative embodiment of the present invention the power supply is not moved directly to 3V but first to a lower voltage, for example 1V. If electrolysis does not occur at this voltage the start pulse will just be repeated with a slightly higher voltage. This will be iteratively repeated until electrolysis and therefore a gas formation is achieved. The voltage at which the gas formation starts may be higher or lower depending on several circumstances as the surface of the electrodes for example a significant roughness, whether a double layer of charge forms on the electrode, conductivity of the fluid, or possibly whether a partially insulating layer forms on the electrode with an associated voltage drop.

Figure 7 shows exemplarily a possible design of a trigger block 23 according to figure 6. For a person skilled in the art it will be obvious that the details of this trigger block 23 can be substituted with a plurality of alternatives known in the art.

In this special embodiment the start pulse is applied to all of the trigger blocks 23 and its action is to pull the input of a comparator 24 to 0V. This causes the output of the comparator 24 to go high which connects the IN terminal 25 to the comparator 24 (which is also initially at 0V) and the Output 26 to 0V. When the start pulse goes low, the input voltage IN is the voltage caused by the potential divider of the fluid resistance R_{FL} and the resistance R. As it drops as the gas bubble evolves it reaches the switch point of the comparator for example by -1V and causes the output OUT of the comparator to go low. This turns off the connection of OUT to 0V so the next stage trigger can function and it also pulls IN high to 3V to stop electrolysis at the input. The input to the comparator is also pulled to -3V to ensure the comparator output remains low.

The comparator can easily be constructed using standard techniques. A technique used often in LTPS circuitry is to use an inverter and capacitor in switched mode to enable the -1V reference voltage to be stored as the switch point of the inverter.

## Claims

1. Method for analyzing one or more aqueous samples for the presence, amount or identity of one or more target molecules in the samples, comprising the steps of
evolving gas by a gas evolving means by electrolysis of the water based sample solution (30) with a voltage of ≥1,2V and ≥10V, thereby
washing away and/ or removing unbound target molecules and/ or redundant sample fluid from a capture site (10), whereby only the hydrogen is used within the gas evolving means.

2. The method according to claim 1, whereby the hydrogen evolving part of the gas evolving means is located in a distance of ≥ 0mm and ≤5 mm from the capture site (10).

3. The method according to any of the claims 1 to 2, whereby the gas evolving means comprises at least one pair of electrodes.

4. The method according to any one of claims 1 to 3, whereby the oxygen is held in solution.

5. The method according to any one of claims 1 to 4, whereby the oxygen is evolved at a remote part of the device.

## Patentansprüche

1. Verfahren zur Analyse von einer oder mehreren wässrigen Proben auf die Anwesenheit, die Menge oder Identität von einem oder mehreren Zielmolekülen in den Proben, wobei das Verfahren die Folgenden Schritte umfasst:
Entwickeln von Gas durch ein Gas entwickelndes Mittel mittels Elektrolyse der Probenlösung auf Wasserbasis (30) mit einer Spannung von ≥ 1,2 V und ≤ 10 V, und **dadurch**
Auswaschen und/oder Entfernen von ungebundenen Zielmolekülen und/oder redundantem Probenfluid aus einer Fangstelle (10), wodurch nur der Wasserstoff innerhalb des Gas entwickelnden Mittels genutzt wird.

2. Verfahren nach Anspruch 1, wobei sich der Wasserstoff entwickelnde Teil des Gas entwickelnden Mittels in einem Abstand von ≥ 0 mm und ≤ 5 mm von der Fangstelle (10) befindet.

3. Verfahren nach einem der Ansprüche 1 bis 2, wobei das Gas entwickelnde Mittel mindestens ein Elektrodenpaar umfasst.

4. Verfahren nach einem der Ansprüche 1 bis 3, wobei der Sauerstoff in Lösung gehalten wird.

5. Verfahren nach einem der Ansprüche 1 bis 4, wobei der Sauerstoff bei einem entfernten Teil der Vorrichtung entwickelt wird.

## Revendications

1. Procédé pour analyser un ou plusieurs échantillons aqueux pour déterminer la présence, la quantité ou l'identité d'une ou plusieurs molécules cibles dans les échantillons, comprenant les étapes consistant à :
dégager un gaz par un moyen de dégagement de gaz par électrolyse de la solution d'échantillon à base d'eau (30) avec une tension supérieure ou égale à 1,2 V et inférieure ou égale à 10 V, et de ce fait
laver et/ou enlever les molécules cibles non liées et/ou le fluide d'échantillon redondant hors d'un site de capture (10), de telle manière que seulement de l'hydrogène soit utilisé à l'intérieur du moyen de dégagement de gaz.

2. Procédé selon la revendication 1, dans lequel la partie de dégagement d'hydrogène du moyen de dégagement de gaz est située à une distance supérieure ou égale à 0 mm et inférieure ou égale à 5 mm du site de capture (10).

3. Procédé selon l'une quelconque des revendications 1 et 2, dans lequel le moyen de dégagement de gaz comprend au moins une paire d'électrodes.

4. Procédé selon l'une quelconque des revendications 1 à 3, dans lequel de l'oxygène est maintenu dans la solution.

5. Procédé selon l'une quelconque des revendications 1 à 4, dans lequel de l'oxygène est dégagé à une partie distante du dispositif.
